# EUROPEAN PATENT APPLICATION

(11) **EP 2 518 086 A1**
(43) Date of publication of application: **31.10.2012**
(21) Application number: 10839480.0
(22) Date of filing: 22.12.2010
(51) Int. Cl.: C07K 19/00, A61K 51/00, A61P 35/00, C07K 14/47, C12P 21/02, C12Q 1/04, G01T 1/161, C12N 15/09

(54) **RADIOLABELED COMPOUND DIRECTABLE IN VIVO TO TARGET TISSUE AND USE THEREOF**

(30) Priority: 25.12.2009 JP 2009295135
(71) Applicant: Riken, Wako-shi, Saitama 351-0198 (JP)
(72) Inventor: WATANABE, Yasuyoshi, Kobe-shi Hyogo 650-0047 (JP); HASEGAWA, Koki, Kobe-shi Hyogo 650-0047 (JP)
(74) Representative: Ladendorf, Oliver
(86) International application number: PCT/JP2010/073189
(87) International publication number: WO 2011/078250

(57) **Abstract**

The present invention provides a clinically usable radiolabeled compound that is precisely directable in vivo to a target tissue. The compound of the present invention has a first polypeptide or an analogue thereof, and a second polypeptide bonded to an N-terminus of the first polypeptide or the analogue thereof. In the compound, the first polypeptide is a polypeptide that specifically binds with a protein expressed in a target tissue, the second polypeptide has an amino acid region that has a high affinity for a radioactive metal nuclide, and the radioactive metal nuclide that has a high affinity for the amino acid region is held in the amino acid region.

SEQUENCE LISTING

<110> RIKEN

<120> A radio-labeled compound directed to a target tissue
      in a living organism and use thereof

<130> RK1685

<150> JP 2009-295135
<151> 2009-12-25

<160> 22

<170> PatentIn Ver. 2.1

<210> 1
<211> 4
<212> PRT
<213> Artificial Sequence

<220>
<223> Description of Artificial Sequence: N-terminal region
      of human albumin

<400> 1
Asp Ala His Lys
  1

<210> 2
<211> 4
<212> PRT
<213> Artificial Sequence

<220>
<223> Description of Artificial Sequence: N-terminal region
      of rat albumin

<400> 2
Glu Ala His Lys
  1

<210> 3
<211> 4
<212> PRT
<213> Artificial Sequence

<220>
<223> Description of Artificial Sequence: N-terminal region
      of bovine albumin

<400> 3
Asp Thr His Lys
  1

<210> 4
<211> 4
<212> PRT
<213> Artificial Sequence

<220>

<221> unsure
<222> (1)...(1)
<223> Xaa is Met, Asp or Glu.

<220>
<221> unsure
<222> (2)...(2)
<223> Xaa is not Pro.

<220>
<223> Description of Artificial Sequence: radioactive
      metal binding

<400> 4
Xaa Xaa His Lys
  1

<210> 5
<211> 6
<212> PRT
<213> Artificial Sequence

<220>
<223> Description of Artificial Sequence: partial sequence
      of P-type ATPase

<400> 5
Met Thr Cys Ala Asn Cys
  1               5

<210> 6
<211> 6
<212> PRT
<213> Artificial Sequence

<220>
<221> unsure
<222> (2)...(2)
<223> Xaa is Thr, Ser, His or Asn.

<220>
<221> unsure
<222> (4)...(4)
<223> Xaa is Ala, Asn, Gln, Ser, Asp, Gly or Glu.

<220>
<221> unsure
<222> (5)...(5)
<223> Xaa is Gly, Ser, Ala, His, Thr, Asn, Gln or Glu.

<220>
<223> Description of Artificial Sequence: radioactive
      metal binding

<400> 6
Met Xaa Cys Xaa Xaa Cys
  1               5

<210> 7
<211> 10
<212> PRT
<213> Artificial Sequence

<220>
<223> Description of Artificial Sequence: radioactive
      metal binding

<400> 7
Asp Ala His Lys Met Thr Cys Ala Gly Cys
  1               5                  10


<210> 8
<211> 11
<212> PRT
<213> Artificial Sequence

<220>
<223> Description of Artificial Sequence: radioactive
      metal binding

<400> 8
Asp Ala His Lys Gly Met Thr Cys Ala Asn Cys
  1               5                  10


<210> 9
<211> 16
<212> PRT
<213> Artificial Sequence

<220>
<223> Description of Artificial Sequence: radioactive
      metal binding

<400> 9
Asp Ala His Lys Met Thr Cys Ala Gly Cys Met Thr Cys Ala Asn Cys
  1               5                  10                  15


<210> 10
<211> 17
<212> PRT
<213> Artificial Sequence

<220>
<223> Description of Artificial Sequence: radioactive
      metal binding

<400> 10
Asp Ala His Lys Gly Met Thr Cys Ala Gly Cys Met Thr Cys Ala Asn
  1               5                  10                  15

Cys


<210> 11
<211> 10
<212> PRT
<213> Artificial Sequence

<220>
<223> Description of Artificial Sequence: radioactive
      metal binding

<400> 11

Asp Thr His Lys Met Thr Cys Ala Gly Cys
  1               5                   10



<210> 12
<211> 11
<212> PRT
<213> Artificial Sequence

<220>
<223> Description of Artificial Sequence: radioactive
      metal binding

<400> 12
Asp Thr His Lys Gly Met Thr Cys Ala Asn Cys
  1               5                   10



<210> 13
<211> 16
<212> PRT
<213> Artificial Sequence

<220>
<223> Description of Artificial Sequence: radioactive
      metal binding

<400> 13
Asp Thr His Lys Met Thr Cys Ala Gly Cys Met Thr Cys Ala Asn Cys
  1               5                   10                  15



<210> 14
<211> 17
<212> PRT
<213> Artificial Sequence

<220>
<223> Description of Artificial Sequence: radioactive
      metal binding

<400> 14
Asp Thr His Lys Gly Met Thr Cys Ala Gly Cys Met Thr Cys Ala Asn
  1               5                   10                  15

Cys




<210> 15
<211> 10
<212> PRT
<213> Artificial Sequence

<220>
<223> Description of Artificial Sequence: radioactive
      metal binding

<400> 15
Glu Ala His Lys Met Thr Cys Ala Gly Cys
  1               5                   10



<210> 16
<211> 11

<212> PRT
<213> Artificial Sequence

<220>
<223> Description of Artificial Sequence: radioactive
      metal binding

<400> 16
Glu Ala His Lys Gly Met Thr Cys Ala Asn Cys
  1               5                  10


<210> 17
<211> 16
<212> PRT
<213> Artificial Sequence

<220>
<223> Description of Artificial Sequence: radioactive
      metal binding

<400> 17
Glu Ala His Lys Met Thr Cys Ala Gly Cys Met Thr Cys Ala Asn Cys
  1               5                  10                  15


<210> 18
<211> 17
<212> PRT
<213> Artificial Sequence

<220>
<223> Description of Artificial Sequence: radioactive
      metal binding

<400> 18
Glu Ala His Lys Gly Met Thr Cys Ala Gly Cys Met Thr Cys Ala Asn
  1               5                  10                  15

Cys



<210> 19
<211> 11
<212> PRT
<213> Artificial Sequence

<220>
<223> Description of Artificial Sequence: partial sequence
      of Cu, Zn superoxide dismutase

<400> 19
His Gly Asp His Met His Asn His Asp Thr Lys
  1               5                  10


<210> 20
<211> 12
<212> PRT
<213> Artificial Sequence

<220>
<223> Description of Artificial Sequence: radioactive
      metal binding

<400> 20
Asp Ala His Lys Phe Cys Phe Trp Lys Thr Cys Thr
  1               5                  10

<210> 21
<211> 15
<212> PRT
<213> Artificial Sequence

<220>
<223> Description of Artificial Sequence: radioactive
      metal binding

<400> 21
Asp Ala His Lys Arg Pro Lys Pro Gln Gln Trp Phe Trp Leu Leu
  1               5                  10                  15

<210> 22
<211> 43
<212> PRT
<213> Artificial Sequence

<220>
<223> Description of Artificial Sequence: radioactive
      metal binding

<400> 22
Asp Ala His Lys His Gly Glu Gly Thr Phe Thr Ser Asp Leu Ser Lys
  1               5                  10                  15

Gln Met Glu Glu Glu Ala Val Arg Leu Phe Ile Glu Trp Leu Lys Asn
             20                  25                  30

Gly Gly Pro Ser Ser Gly Ala Pro Pro Pro Ser
         35                  40

## Description

### Technical Field

The present invention relates to a radiolabeled compound that is directable in vivo to a target tissue and use thereof.

### Background Art

Radiotherapy is therapy using radiation mainly for treatment of a cancer (malignancy). The radiotherapy is used alone or in combination with chemotherapy, hormone therapy, or the like. In recent years, various radioligands for in vivo treatment and diagnosis have been investigated. For example, a radioactive drug (Zevalin (Registered Trademark)) using a radiolabeled antibody has been developed for the purpose of treatment of a cancer, which radiotherapy is targeted at.

A known example of a technique using a radiolabeled compound administered into a living body is positron emission tomography (PET). PET is one of "molecular imaging" techniques which are capable of analyzing the behavior of a molecule of interest. PET is considered as being useful for finding of various diseases at their early stages and short-time development of effective medicine with little adverse effect. PET is a technique which captures a tomographic image using gamma rays emitted when a positron collides with an electron. PET is carried out by administering a labeled-molecule of interest (so-called molecular probe) to a living body. The radionuclide labeled to the molecule decays into another nucleus while emitting a positron, and the positron collides with a neighboring electron to emit gamma rays being detected. This allows quantitative evaluation of the site where the molecule of interest exists. The PET is widely used as a clinical molecular imaging technique. For example, PET using ¹⁸F-labeled fluorodeoxyglucose is used for health check relevant to cancer.

A radioactive drug which is administered into a living body can directly irradiate a tumor cell of interest with radiation. This provides a novel anti-tumor effect. Furthermore, PET is an excellent molecular imaging technique used for development of medicine and clinical diagnosis. As has been described, such techniques using a radiolabeled compound administered into a living body are very promising.

### Citation List

### Non Patent Literature 1

Lewis et al., Bioconjugate Chemistry (1994), 5(6): 565-576

### Summary of Invention

### Technical Problem

Zevalin is a compound that is labeled with a radioactive metal nuclide emitting a beta ray via a chelator that has been covalently bound to an antibody against a protein of interest. Also in a case where a biopolymer such as an antibody or a protein is radiolabeled for application to PET, a method is adopted in which (i) a chelator (e.g., DOTA (1,4,7,10-tetraazacyclododecane-N,N',N",N"'-tetraacetic acid)) is introduced into the biopolymer via a linker and (ii) a metal nuclide is combined with the chelator (see, for example, Non Patent Literature 1).

However, according to the technique described in Non Patent Literature 1, the chelator non-specifically reacts with the biopolymer. Accordingly, the introduction of the chelator into an active site of the biopolymer causes a decline in activity of the biopolymer. Further, such a technique requires chemical modification of a biopolymer with a chelator. This requires time and effort. Furthermore, a chelator to be used is a substance that does not originally exists in a living body. Accordingly, it is necessary to evaluate safety of the chelator itself in the living body in order to use a radiolabeled biopolymer as a drug or the like.

The present invention was attained in view of the above problems, and an object of the present invention is to provide a radiolabeled compound that is clinically usable in a living body without chemical modification with a chelator.

### Solution to Problem

In order to attain the above object, the inventors of the present invention found, as a result of diligent studies from a unique perspective, that a specific region of a protein, which is one of biopolymers, has a binding capacity suitable for allowing a radioactive metal nuclide to direct to a target tissue in a living body. Based on this finding, the inventors of the present invention accomplished the present invention.

A compound of the present invention has: a first polypeptide or an analogue thereof; and a second polypeptide bonded to an N-terminus of the first polypeptide or the analogue thereof, the first polypeptide being a polypeptide that specifically binds with a protein expressed in a target tissue, the second polypeptide having an amino acid region that has a high affinity for a radioactive metal nuclide, and the radioactive metal nuclide that has a high affinity for the amino acid region being held in the amino acid region.

According to the arrangement, the compound of the present invention can be delivered to a target site in a living body while holding a radioactive metal nuclide.

In the compound of the present invention, the amino acid region is preferably a fragment of a naturally-occurring protein that exists in a living body to which the compound is applied. The protein expressed in the target tissue is preferably a receptor protein for the first polypeptide. According to the arrangement, the compound of the present invention has a low risk of being toxic to the living body to which the compound of the present invention has been administered.

In the compound of the present invention, the amino acid region preferably contains X1-His where X1 represents any amino acid other than Pro, and more preferably contains X2-X1-His or X2-X1-His-Lys where X1 represents any amino acid other than Pro and X2 represents Met, Asp or Glu. In a case where the compound of the present invention has the amino acid region with such an arrangement, a complex with radioactive metal ⁶⁴Cu is successfully formed. This allows ⁶⁴Cu, which has a relatively long half-life, to be used as the radioactive metal nuclide.

In the compound of the present invention, the amino acid region may contain Met-X3-Cys-X4-X5-Cys where X3 represents Thr, Ser, His or Asn, X4 represents Ala, Asn, Gln, Ser, Asp, Gly or Glu, and X5 represents Gly, Ser, Ala, His, Thr, Asn, Gln or Glu. Further, in the compound of the present invention, the amino acid region may contain His-Gly-Asp-His-Met-His-Asn-His-Asp-Thr-Lys.

In the compound of the present invention, the second polypeptide can be DAH, DAHK, DTH, DTHK, EAH, EAHK, MTCANC, DAHKMTCAGC, DAHKGMTCANC, DAHKMTCAGCMTCANC, DAHKGMTCAGCMTCANC, DTHKMTCAGC, DTHKGMTCANC, DTHKMTCAGCMTCANC, DTHKGMTCAGCMTCANC, EAHKMTCAGC, EAHKGMTCANC, EAHKMTCAGCMTCANC, EAHKGMTCAGCMTCANC, or HGDHMHNHDTK.

In the compound of the present invention, the amino acid region preferably constitutes an N-terminus portion of the second polypeptide. Further, in the compound of the present invention, the second polypeptide may be made up of 3 to 15 amino acids. According to the arrangement, the compound of the present invention can easily pass through a blood-brain barrier. Accordingly, the compound of the present invention can be applied even in a case where it is to be delivered to a head.

A method of the present invention for producing a compound includes the step of bonding a second polypeptide to an N-terminus of a first polypeptide or an analogue thereof, the first polypeptide being a polypeptide that specifically binds with a protein expressed in a target tissue, the second polypeptide having an amino acid region that has a high affinity for a radioactive metal nuclide, and, the method further including the step of causing the radioactive metal nuclide that has a high affinity for the amino acid region to be held in the amino acid region.

In the method of the present invention for producing a compound, the step of bonding the second polypeptide may be carried out by synthesizing the second polypeptide to the N-terminus of the first polypeptide or the analogue thereof by solid-phase peptide synthesis or may be carried out by generating a fusion protein between the first polypeptide and the second polypeptide by a genetic engineering method. In a case where the genetic engineering method is adopted, an amino acid region of interest can be bonded in advance to the N-terminus of the first polypeptide. This can save time and effort of chemically modifying a chelator after a protein is prepared. Further, a site to which the amino acid region of interest is to be bonded is easily restricted to a specific site. Furthermore, use of such a method makes labeling of a membrane protein on a cell surface easy, thereby making labeling of a cell easy.

A composition of the present invention for allowing a radioactive metal nuclide to direct to a target tissue in a living body, contains a compound having: a first polypeptide or an analogue thereof; and a second polypeptide bonded to an N-terminus of the first polypeptide or the analogue thereof, the first polypeptide being a polypeptide that specifically binds with a protein expressed in a target tissue, and the second polypeptide having an amino acid region that has a high affinity for the radioactive metal nuclide. The composition of the present invention may further contain the radioactive metal nuclide that has a high affinity for the amino acid region.

The composition of the present invention may be used for diagnosis using positron emission tomography or may be used for radiotherapy.

A method of the present invention for capturing a positron emission tomography image in a living body includes the step of detecting, from a living body, gamma-ray activity generated based on a positron emitted by a compound administered into the living body, the compound having a first polypeptide or an analogue thereof, and a second polypeptide bonded to an N-terminus of the first polypeptide or the analogue thereof, and in the compound, the first polypeptide being a polypeptide that specifically binds with a protein expressed in a target tissue to be measured, the second polypeptide having an amino acid region that has a high affinity for a radioactive metal nuclide, and the radioactive metal nuclide that has a high affinity for the amino acid region being held in the amino acid region.

A method of the present invention for screening for a compound for use in radiotherapy includes the step of detecting, from a living body, gamma-ray activity generated based on a positron emitted by a compound administered into the living body, the candidate compound having a first polypeptide or an analogue thereof, and a second polypeptide bonded to an N-terminus of the first polypeptide or the analogue thereof, in the candidate compound, the first polypeptide being a polypeptide that specifically binds with a protein expressed in a target tissue to be measured, the second polypeptide having an amino acid region that has a high affinity for a radioactive metal nuclide, and the radioactive metal nuclide that has a high affinity for the amino acid region being held in the amino acid region.

A method of the present invention for detecting a protein of interest in a sample includes the steps of: incubating the sample with a compound; and detecting gamma-ray activity generated based on a positron emitted from the compound, the compound having a first polypeptide or an analogue thereof, and a second polypeptide bonded to an N-terminus of the first polypeptide or the analogue thereof, in the compound, the first polypeptide being a polypeptide that specifically binds with a membrane protein of interest, the second polypeptide having an amino acid region that has a high affinity for a radioactive metal nuclide, and the radioactive metal nuclide that has a high affinity for the amino acid region being held in the amino acid region.

A method of the present invention for labeling a cell includes the steps of: incubating the cell with a compound; and detecting gamma-ray activity generated based on a positron emitted from the compound, the compound having a first polypeptide or an analogue thereof, and a second polypeptide bonded to an N-terminus of the first polypeptide or the analogue thereof, in the compound, the first polypeptide being a polypeptide that specifically binds with a membrane protein expressed in the cell to be labeled, the second polypeptide having an amino acid region that has a high affinity for a radioactive metal nuclide, and the radioactive metal nuclide that has a high affinity for the amino acid region being held in the amino acid region.

Another method of the present invention for labeling a cell includes the steps of: transforming the cell with use of a polynucleotide encoding a fusion protein between a membrane protein and a second polypeptide bonded to an N-terminus of the membrane protein; and causing a radioactive metal nuclide to be held in a second polynucleotide, the second polypeptide having an amino acid region that has a high affinity for the radioactive metal nuclide.

Additional objects, advantages and novel features of the invention will be set forth in part in the description which follows, and in part will become apparent to those skilled in the art upon examination of the following or may be learned by practice of the invention.

### Advantageous Effects of Invention

According to the present invention, a protein, which is a biopolymer, can be radiolabeled without the need for chemical modification with a low molecular compound (chelator) or the like whose toxicity is unknown, unlike the conventional arts. Moreover, according to radiolabeling using a chelator such as DOTA, it was extremely difficult to introduce a desired number of radiolabels into a desired site. However, use of the present invention allows an N-terminus of a protein to be specifically radiolabeled and allows the number of metal nuclides labeling the protein to be restricted.

### Brief Description of Drawings

Fig. 1

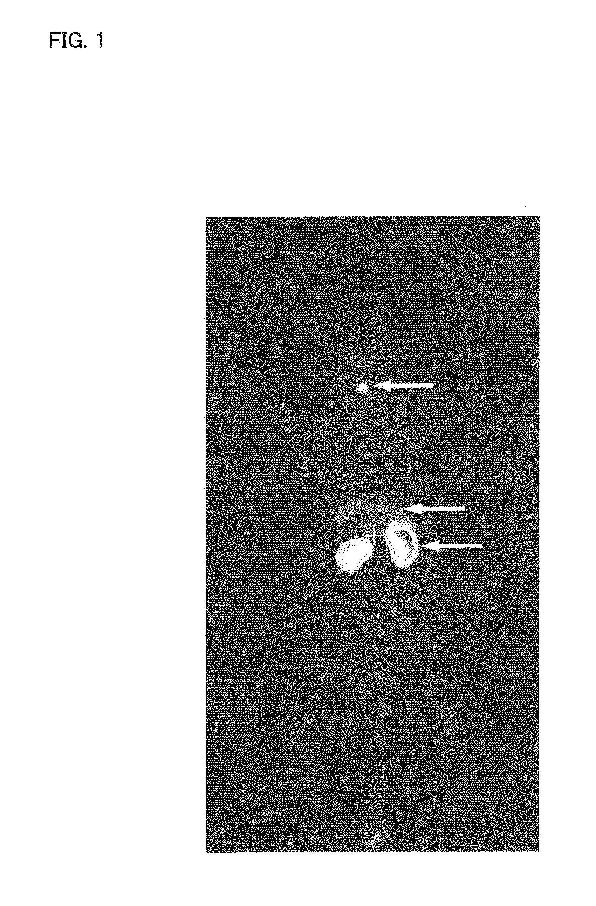

   Fig. 1 is a drawing showing a PET image of the whole body of a rat to which a compound of the present invention has been administered.
Fig. 2

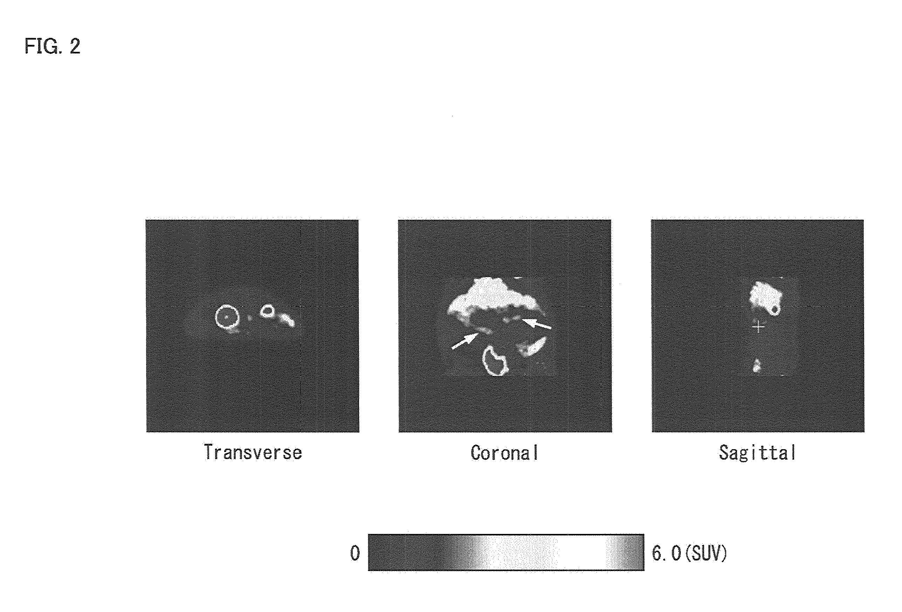

   Fig. 2 is a drawing showing a PET image of an abdomen of the rat to which the compound of the present invention has been administered.
Fig. 3

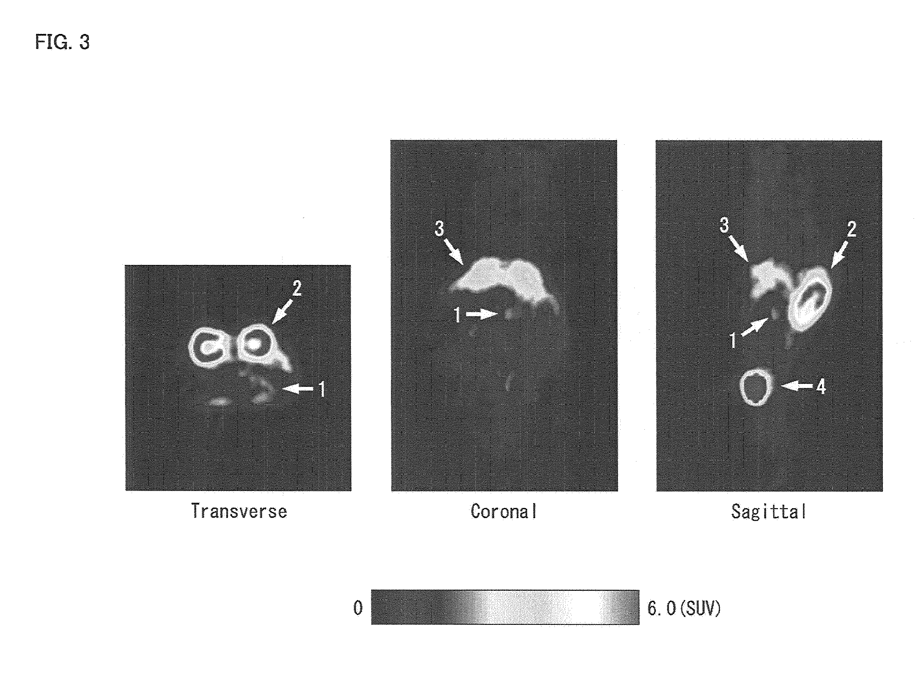

   Fig. 3 is a drawing showing a PET image of the whole body of a nude mouse to which a compound of the present invention has been administered.

### Description of Embodiments

An embodiment of the present invention is described below in detail.

### [1] Compound of the Present Invention

The present invention provides a compound which has a first polypeptide or an analogue thereof and a second polypeptide bonded to an N-terminus of the first polypeptide or the analogue thereof.

### [1-1] Polypeptide and Polynucleotide

The term "polypeptide" used herein is interchangeable with "peptide" or "protein". A "fragment" of a polypeptide refers to a part of the polypeptide. A polypeptide may be isolated from a natural source, may be chemically synthesized, or may be generated by a genetic engineering method.

The term "isolated" polypeptide refers to a polypeptide extracted from a natural environment. For example, a recombinant polypeptide expressed in a host cell (transformant) is deemed as being isolated, as with a natural or recombinant polypeptide that is substantially purified by any appropriate technique.

A generated polypeptide can be easily purified by affinity chromatography using an antibody that can specifically bind with the polypeptide. A polypeptide of interest can be purified not only by using the antibody affinity chromatography, but also by using a wide variety of known protein purification techniques (e.g., ammonium sulphate fractionation, solvent precipitation, immunoprecipitation, gel filtration, ion exchange chromatography, hydrophobic chromatography, isoelectric precipitation, electrofocusing, electrophoresis, etc.) alone or in combination. The antibody used in the affinity chromatography may be a polyclonal antibody or may be a monoclonal antibody, and can be produced by a well known method in the art (e.g., a peptide antibody).

In the present invention, the polypeptide may have an additional polypeptide for the purpose of simplifying a purification procedure. Examples of the additional polypeptide include a polypeptide tagged with His, Myc, Flag or the like, and GST. Such an additional polypeptide can be successively cleaved by a known method in the art after purification.

A person skilled in the art can easily obtain a nucleotide sequence of a polynucleotide encoding a polypeptide of interest on the basis of an amino acid sequence of the polypeptide of interest. The term "polynucleotide" used herein is interchangeable with "gene", "nucleic acid" or "nucleic acid molecule", and refers to a nucleotide polymer. A "polynucleotide" in which several or several tens of nucleotides are bonded to each other is interchangeable with "oligonucleotide". The term "nucleotide sequence" used herein is interchangeable with "nucleic acid sequence" or "base sequence", and is expressed by a sequence of deoxyribonucleotides (abbreviated as A, G, C, and T). A "polynucleotide having a nucleotide sequence represented by SEQ ID NO: X or a fragment thereof" refers to a polynucleotide having a sequence of deoxynucleotides A, G, C and/or T represented by SEQ ID NO: X or a part thereof.

As used herein, a polynucleotide can exist in the form of DNA (e.g., cDNA or genome DNA) or in the form of RNA (e.g., mRNA). DNA may be double-strand DNA or may be single-strand DNA. The single-strand DNA or RNA can be a coding strand (known also as a sense strand) or can be a noncoding strand (known also as an antisense strand).

As used herein, a polynucleotide may have a sequence such as a sequence of an untranslated region or a vector sequence. Further, a polynucleotide may be linked in frame to a polynucleotide encoding a tag label at the 5' end or 3' end thereof. This allows a polypeptide encoded by the polynucleotide to have the aforementioned additional polypeptide.

### [1-2] First Polypeptide

In the compound of the present invention, the first polypeptide bears a function of causing the compound of the present invention to direct to a target tissue in a living body. A tissue in which a protein that specifically binds with the first polypeptide is expressed is used as the target tissue. This allows the compound of the present invention to be delivered to a desired site in a living body. That is, the first polypeptide is a polypeptide that specifically binds with a protein expressed in the target tissue.

The protein that specifically binds with the first polypeptide is, for example, a receptor for the first polypeptide, but is not limited to this. In a case where it is desired that a site to which the compound of the present invention is delivered be limited to a specific site in a living body, a protein that is specifically expressed in the specific site is selected as the protein. A preferable example of such a protein is a membrane protein, but such a protein may be an extracellular matrix protein that is tissue-specifically expressed. A person skilled in the art who has selected a tissue to which the compound of the present invention is to be directed can easily select, based on a common technical knowledge in the art, a protein that is specifically expressed in the tissue and can easily select a polypeptide that specifically binds with the protein thus selected. That is, a person skilled in the art can easily select, based on a common technical knowledge in the art, the first polypeptide in accordance with the target tissue.

As used herein, an analogue of the first polypeptide refers to a compound that has a binding capacity of specifically binding to a receptor for the first polypeptide but has a chemical structure different from the first polypeptide. That is, a protein that specifically binds with the first polypeptide can also be a protein that specifically binds with an analogue of the first polypeptide.

In an Example described later, the present invention is demonstrated by using, as an example, octreotide which is one of somatostatin analogues. Note, however, that the analogue of the first polypeptide is not limited to this. Note also that the somatostatin analogue is not limited to a specific one, provided that it is a compound which has a binding capacity of specifically binding to at least one of five sub-types (SSTR1 to 5) of somatostatin receptors. Examples of the somatostatin analogue include octreotide and an octreotide analogue. Examples of the octreotide analogue include [Tyr³]-Octreotide, Vapreotide, Lanreotide, [Tyr³]-Octreotate, and [Tyr³,Tyr⁸]-Octreotide (see Storch et al., The Journal of Nuclear Medicine, Vol.46, No.9, 2005, Reubi et al., European Journal of Nuclear Medicine, Vol.27, No.3, 2000). The "analogue" in the present invention also encompasses compounds obtained by subjecting the foregoing compounds to chemical modification (e.g., modification with polyethylene glycol (PEG)). As described above, a person skilled in the art can select, based on a common technical knowledge in the art, the first polypeptide depending on the target tissue. Further, in a case where it is intended that information in a living body be observed for a relatively long period of time, a person skilled in the art can easily select, based on a common technical knowledge in the art, an analogue having a longer physiologic half-life (i.e., relatively slow metabolism in or excretion from a living body).

Other examples of the first polypeptide include exenatide, which directly binds with a receptor for glucagon-like peptide 1 (GLP-1), spantide, which is a substance P receptor antagonist, epidermal growth factor (EGF), and interferon (IFN). The amino acid sequences of EGF, IFN, etc. are well-known in the art, and a person skilled in the art can easily obtain such amino acid sequences.

### [1-3] Second Polypeptide

In the compound of the present invention, the second polypeptide bears a function of holding a radioactive metal nuclide until the compound of the present invention reaches the target tissue. The second polypeptide can successfully execute such a function since the second polypeptide has an amino acid region that has a high affinity for the radioactive metal nuclide. That is, the second polypeptide is a polypeptide having an amino acid region that has a high affinity for a radioactive metal nuclide. The second polypeptide is preferably a fragment of a biomolecule (i.e., a naturally-occurring protein). This reduces a risk that the compound of the present invention to be administered into a living body becomes toxic to the living body.

In an embodiment, the compound of the present invention is a radiolabeled compound that is directable to a target tissue in a living body. In the present embodiment, a radioactive metal nuclide is held in the amino acid region. With such an arrangement, the radioactive metal nuclide can be delivered to the target tissue. Accordingly, the compound of the present invention is very useful as a drug for use in radiotherapy. Furthermore, the compound of the present invention can provide information based on gamma-ray activity produced by the radioactive metal nuclide (e.g., information on dynamics of a substance in a living body). Accordingly, the compound of the present invention used as a molecular probe (so-called PET probe) for positron emission tomography is very useful for PET image diagnosis.

As described above, the compound of the present invention has the first polypeptide or an analogue thereof and the second polypeptide bonded to an N-terminus of the first polypeptide or the analogue thereof. In a case where the compound of the present invention is used for radiotherapy, it is only necessary that the radioactive metal nuclide held in the second polypeptide be delivered to a target tissue. Accordingly, in this case, the first polypeptide is not limited to a specific one, provided that it is a polypeptide that specifically binds with a protein expressed in the target tissue. It is important that the second polypeptide be bonded to a site that does not inhibit the binding between the first polypeptide and the protein. Accordingly, the second polypeptide is bonded to the N-terminus of the first polypeptide.

As described above, the second polypeptide has an amino acid region that has a high affinity for a radioactive metal nuclide. Such an amino acid region is preferably a fragment of a naturally-occurring protein that exists in a living body to which the compound of the present invention is applied. With such an arrangement, it is possible to greatly reduce a risk that the present invention becomes toxic to the living body. The second polypeptide having such an arrangement may be a naturally-occurring protein itself that exists in a living body to which the compound of the present invention is applied.

The second polypeptide may have a plurality of amino acid regions each of which has a high affinity for a radioactive metal nuclide. In this case, the plurality of amino acid regions may be identical to each other or may be different from each other.

In one aspect, the amino acid region preferably contains X1-His where X1 represents any amino acid other than Pro, and more preferably contains X2-X1-His or X2-X1-His-Lys where X1 represent any amino acid other than Pro and X2 represents Met, Asp or Glu. In a case where the second polypeptide has an amino acid region with such an arrangement, ⁶⁴Cu, which has a relatively long half-life, can be used as the radioactive metal nuclide. This allows the compound of the present invention to be suitably applied as a PET probe. The amino acid region with such an arrangement is especially suitably used in a case where a bivalent metal ion is to be bonded. Examples of the radioactive metal nuclide that has a high affinity for the amino acid region with such an arrangement include ⁶⁴Cu, ⁶⁵Zn, ⁷²Zn, ⁵⁶Co, ⁵⁷Co, ⁵⁸Co, ⁶⁰Co, ¹⁰⁹Cd, ¹¹³Cd, ⁵⁶Ni, ⁵⁹Ni, and ⁶³Ni, each of which can be suitably used. Another example of an amino acid region that can be used in a case where a bivalent metal ion is to be bonded is one that contains His-Gly-Asp-His-Met-His-Asn-His-Asp-Thr-Lys. Examples of a radioactive metal nuclide that has a high affinity for the amino acid region with such an arrangement include ⁶⁴Cu, ⁶⁵Zn, ⁷²Zn, ⁵⁶Ni, ⁵⁹Ni, and ⁶³Ni, each of which can be suitably used.

In another aspect, the amino acid region preferably contains Met-X3-Cys-X4-X5-Cys where X3 represents Thr, Ser, His or Asn, X4 represents Ala, Asn, Gln, Ser, Asp, Gly or Glu, and X5 represents Gly, Ser, Ala, His, Thr, Asn, Gln or Glu, and more preferably contains Met-Thr-Cys-Ala-Asn-Cys. The amino acid region with such an arrangement is especially suitably used in a case where a monovalent metal ion is to be bonded. This allows the compound of the present invention to be suitably applied as a PET probe. Examples of the radioactive metal nuclide that has a high affinity for the amino acid region with such an arrangement include ⁶⁴Cu, ⁶⁵Zn, ⁷²Zn, ⁵⁶Co, ⁵⁷Co, ⁵⁸Co, ⁶⁰Co, ¹⁰⁹Cd, ¹¹³Cd, and ¹⁹⁴Hg, each of which can be suitably used.

In a case where the compound of the present invention is to be delivered to a head (especially into a brain), the second polypeptide preferably has a short length, and is more preferably made up of 3 to 15 amino acids, in light of a capacity for passing through a blood-brain barrier. In this case, the second polypeptide may be a peptide made up of the amino acid region.

In the compound of the present invention, it is only necessary that the amino acid region be contained in the second polypeptide. In a case where the amino acid region constitutes an N-terminus portion of the second polypeptide (i.e., the amino acid region is exposed to the N-terminus of the second polypeptide), the radioactive metal nuclide can be more firmly held. This increases quality stability.

Examples of an amino acid region which is suitably used in the compound of the present invention and which has a high affinity for a radioactive metal nuclide include, but are not limited to, DAH, DAHK, DTH, DTHK, EAH, EAHK, MTCANC, DAHKMTCAGC, DAHKGMTCANC, DAHKMTCAGCMTCANC, DAHKGMTCAGCMTCANC, DTHKMTCAGC, DTHKGMTCANC, DTHKMTCAGCMTCANC, DTHKGMTCAGCMTCANC, EAHKMTCAGC, EAHKGMTCANC, EAHKMTCAGCMTCANC, EAHKGMTCAGCMTCANC, and HGDHMHNHDTK. The second polypeptide may be made up of these amino acid regions.

In an Example described later, the present invention is demonstrated by using, as an example, a compound in which the second polypeptide made up of Asp-Ala-His-Lys is bonded to an N-terminus of octreotide which is one of analogues of somatostatin serving as the first polypeptide. The compound of the present invention accumulates in pancreas and hypophysis in which a receptor for the somatostatin is specifically expressed. This shows that radiotherapy to these portions is possible. In another Example, the present invention is demonstrated by using, as an example, a compound in which the second polypeptide made up of Asp-Ala-His-Lys is bonded to an N-terminus of exenatide. The compound of the present invention accumulates in pancreas in which a GLP-1 receptor is highly expressed, as in the Example using octreotide. Similarly, a compound (Asp-Ala-His-Lys-D-Arg-Pro-Lys-Pro-Gln-Gln-D-Trp-Phe-D-Trp-Leu-Leu) in which the second polypeptide made up of Asp-Ala-His-Lys is bonded to an N-terminus of spantide can also be used. Of course, the first polypeptide may have a function as a drug. In this case, a synergetic effect produced by the function of the first polypeptide and the function of the metal nuclide can be expected. Furthermore, the inventors of the present invention produced a compound in which the second polypeptide made up of Asp-Ala-His-Lys-Met-Thr-Cys-Ala-Gly-Cys is bonded to an N-terminus of octreotide or exenatide.

It is known that an amino acid sequence of Asp-Ala-His-Lys exists in human albumin and that a bivalent metal ion is bonded to this region (see Malgorzata Rozga et al., J. Biol. Inorg. Chem. (2007), 12: 913-918, David Bar-Or et al., Biochem. Biophys. Res. Commun. (2001), 284(3): 856-862, Leonard T.R. et al., Biochem. Biophys. Res. Commun. (2007), 357: 543-548, Catherine Harford and Bibudhendra Sarkar, Acc. Chem. Res. (1997), 30: 123-130, Peter J. Sadler et al., Eur. J. Biochem. (1994), 220: 193-200). Further, it is known that an amino acid sequence of His-Gly-Asp-His-Met-His-Asn-His-Asp-Thr-Lys exists in Cu,Zn superoxide dismutase (Cu,ZnSOD) and that a bivalent metal ion is bonded to this region (see J Inorg Biochem. (2008) 102(9): 1700-10, J. Mol. Biol. (2009) 386: 406-418, etc.). Furthermore, it is known that an amino acid sequence of Met-X3-Cys-X4-X5-Cys exists in P-type ATPase (CopA) and that a monovalent metal ion is bonded to this region (see Biochemistry (2005) 44: 13144-13150, J Biol Chem. (2001) 276: 30315-30325, Biochem. Biophys. Res. Comm. (2007) 357: 543-548, Genome Res. (2002) 12: 255-271, J Biol Chem. (2007) 282: 8622-8631, Biochem. J. (2008) 411: 571-579, etc.).

However, these literatures neither describe nor suggest anything about bonding with a radioactive metal nuclide. Moreover, these literatures neither teach nor suggest anything about a radiolabeled compound. In addition, these literatures describe that a bonded bivalent metal ion is easily dissociated. Accordingly, a person skilled in the art cannot easily reach, on the basis of these descriptions, a radiolabeled compound that is clinically usable in a living body. Especially considering that use of a region that is present in a living body can bring about a state in which the region competes with a naturally-occurring protein that exists in the living body so that they inhibit each other's functions, a person skilled in the art cannot easily reach the arrangement of the compound of the present invention. As has been described, a person skilled in the art cannot easily attain, on the basis of the conventional knowledge, a compound that can be delivered to a target site in a living body while holding a radioactive metal nuclide.

As described above, in a radiolabeled compound that is used as a molecular probe for PET or a radioactive drug, a chelator such as DOTA is used. This indicates that a chelator is extremely good in bonding a radioactive metal. The results shown in Examples described later are equivalent to a result obtained by use of a compound produced with the use of DOTA. A person skilled in the art cannot expect that such an excellent effect can be produced without the use of a chelator.

In the first place, an amount of radioactive metal nuclide used when a compound is radiolabeled is small. Accordingly, concentration of the radioactive metal nuclide in a reaction solution is extremely low (For example, 1000-MBq ⁶⁴Cu is 7 ng in weight. Accordingly, in a case where 300-MBq ⁶⁴Cu is dissolved in 100 µL of a reaction solution, concentration of ⁶⁴Cu in the solution is approximately 0.3 µM). Since peptide of approximately 1 to 10 nmole is added thereto and is reacted, radiolabeling reaction is performed under an environment of very low concentration. A person skilled in the art cannot easily expect that labeling reaction of the compound of the present invention successfully proceeds in spite of such an environment of very low concentration. Needless to say, a person skilled in the art cannot easily reach the arrangement (i.e., the arrangement of the present invention) which allows labeling reaction to successfully proceed.

Note that since an object of the present invention is to provide a radiolabeled compound that is directable to a target tissue in a living body, the present invention is not limited to specific polypeptides described in the specification. Note also that a radiolabeled compound obtained by a method other than the method described below is also encompassed within the technical scope of the present invention.

### [2] Method for Producing Compound of the Present Invention

The present invention also provides a method for producing the above compound. The method of the present invention includes the steps of: bonding a second polypeptide to an N-terminus of a first polypeptide or an analogue thereof; and causing a radioactive metal nuclide to be held in the second polypeptide. As described in the above description concerning the compound, the first polypeptide is a polypeptide that specifically binds with a protein expressed in a target tissue, and the second polypeptide has an amino acid region that has a high affinity for the radioactive metal nuclide. That is, the step of causing a radioactive metal nuclide to be held in the second polypeptide is, in other words, a step of causing a radioactive metal nuclide which has a high affinity for the amino acid region to be held in the amino acid region. See [1-2] and [1-3] above as for the first polypeptide and the second polypeptide.

In an embodiment, the step of bonding the second polypeptide is carried out by synthesizing the second polypeptide to the N-terminus of the first polypeptide or an analogue thereof with the use of solid-phase peptide synthesis. In another embodiment, the step of bonding the second polypeptide is carried out by generating a fusion protein between the first polypeptide and the second polypeptide by a genetic engineering method.

In a case where the first polypeptide is a naturally-occurring protein (or a fragment thereof), the compound of the present invention is preferably produced as a fusion protein between the first polypeptide and the second polypeptide by a genetic engineering method. Meanwhile, in a case where the first polypeptide is a synthesized peptide (e.g., a synthetic obtained by modifying a naturally-occurring protein), the compound of the present invention is preferably produced by solid-phase peptide synthesis.

In a case where a compound that is to be delivered to a head (especially into a brain) is produced, the second polypeptide preferably has a short length as described above. In a case where such a short peptide is used as the second polypeptide, the solid-phase peptide synthesis may be used even in a case where the first polypeptide is a naturally-occurring protein (or a fragment thereof).

The polypeptide production using a genetic engineering method may be one using a transformant or may be one using in vitro translation. The transformant (e.g., a prokaryotic cell and a eukaryotic cell) may be one which transiently expresses a polypeptide of interest or may be one which stably expresses the polypeptide of interest. In a case where the polypeptide of interest is transiently expressed, the polypeptide of interest can be speedily prepared. Meanwhile, in a case where the polypeptide of interest is stably expressed, the polypeptide of interest can be prepared in large amounts.

The production using a genetic engineering method can be carried out with the use of a vector into which a polynucleotide encoding the polypeptide of interest is functionally inserted. The term "functionally inserted" used herein refers to insertion into a vector in accordance with appropriate open reading frame and direction. The vector used in the production using a genetic engineering method may be a vector for in vitro translation or may be a vector for use in recombinant expression. A method for producing such a vector can be, for example, a method using plasmid, phase, cosmid, or the like, but is not limited to a specific one.

The vector for use in recombinant expression is not limited to a specific one, and a vector which can be expressed in a host cell can be appropriately selected. Specifically, a promoter sequence for surely expressing a gene (polynucleotide) of interest is appropriately selected depending on the kind of a host cell, and a vector obtained by introducing the promoter sequence thus selected and the gene of interest into a plasmid or the like of various kinds is used as an expression vector. Transformation of the host by the expression vector can also be carried out by a conventional method.

The host cell transformed with the use of the expression vector is cultured. Then, a protein of interest can be collected/ purified from a cultured product or a culture supernatant by a conventional method (e.g., filtration, centrifugal separation, cell breakage, gel filtration chromatography, ion exchange chromatography, etc.).

The expression vector preferably contains a conventionally known selection marker. A method for introducing the expression vector into the host cell, i.e., a transformation method is not limited to a specific one, and a conventionally known method can be suitably used.

A person skilled in the art who attempts the polypeptide production using a genetic engineering method can easily acquire information on amino acid sequence and nucleotide sequence of interest from any sequence database. Further, a person skilled in the art can easily construct a necessary gene library on the basis of the technical common knowledge in the art and can easily prepare a gene having a desired nucleotide sequence from the gene library thus constructed. Furthermore, a person skilled in the art can easily prepare, on the basis of the technical common knowledge in the art, a gene in which a polynucleotide encoding an amino acid region of interest is inserted to a desired site of the gene thus obtained.

As described above, in the compound of the present invention, the amino acid region preferably constitutes the N-terminus portion of the second polypeptide (i.e., the amino acid region is preferably exposed to the N-terminus of the second polypeptide). A person skilled in the art can create the amino acid region so that it constitutes the N-terminus portion of the second polypeptide on the basis of the technical common knowledge in the art. For example, in a case where the solid-phase peptide synthesis is used, amino acids of interest are sequentially synthesized to the N-terminus of the second polypeptide. In a case of the polypeptide production using a genetic engineering method, the amino acid region may be created subsequently to an initiator methionine. In this case, a fusion protein between the first polypeptide and the second polypeptide in which an enzyme site for cleavage is provided on the N-terminus side of the amino acid region is produced, and then a cleavage process is carried out with the use of a predetermined enzyme. Such a cleavage process is well known in the art.

### [3] Composition of the Present Invention

As described above, use of the compound of the present invention allows a radioactive metal nuclide to direct to a target tissue in a living body. Accordingly, the compound of the present invention is especially useful for diagnosis using positron emission tomography and for radiotherapy.

That is, the present invention provides a composition which allows a radioactive metal nuclide to direct to a target tissue in a living body. The composition of the present invention may be prepared in the form of a solution or suspension or may be prepared in the form of a solid suitable for being dissolved or suspended in a liquid (e.g. buffer).

The composition of the present invention contains, as an arrangement necessary for directing a radioactive metal nuclide to a target tissue in a living body, a compound which has a first polypeptide or an analogue thereof and a second polypeptide bonded to an N-terminus of the first polypeptide or the analogue thereof. The first polypeptide is a polypeptide that specifically binds with a protein expressed in the target tissue, and the second polypeptide has an amino acid region that has a high affinity for the radioactive metal nuclide. See [1-2] and [1-3] above as for the first polypeptide and the second polypeptide.

That is, the composition of the present invention may contain a compound in which the radioactive metal nuclide has not been held yet or may contain a compound in which the radioactive metal nuclide has been held. In the former case, the composition of the present invention can be a composition for preparing a radiolabeled compound that is directable to a target tissue in a living body.

The composition of the present invention may be provided in the form of a kit. The term "composition" refers to a substance in which two or more components are contained. The term "kit" refers to a substance in which at least one of the components is contained in other material. As used herein, a "composition containing an effective component" encompasses a "kit containing an effective component". In the case of the form of a kit, the kit contains the compound described later as the effective component, and may further contain a component that can be contained as a composition. That is, the composition of the present invention can be a kit which separately contains a "compound in which a radioactive metal nuclide has not been held yet" and the "radioactive metal nuclide".

The term "kit" used herein refers to a package including a container (e.g. bottle, plate, tube, and dish) containing therein a specific material. The kit preferably has directions for using individual materials. The term "includes (including)" used in the aspect of the kit in the present specification indicates that a material is contained in any one of individual containers constituting the kit. Further, the kit of the present invention may be a package in which a plurality of different compositions are contained. The "directions" may be written or printed on a paper or other kind of a medium. Alternatively, the "directions" may be in the form of a magnetic tape or an electronic medium such as a computer-readable disc or tape and a CD-ROM. Further, the kit of the present invention may include a container containing a diluent, a solvent, a cleaning liquid, or other reagent. Further, the kit of the present invention may include an instrument necessary for application.

### [4] Other Application of the Present Invention

As described above, the compound and composition of the present invention can be used for diagnosis using positron emission tomography due to its capacity for directing a radioactive metal nuclide to a target tissue in a living body. That is, the present invention provides a method for capturing a positron emission tomography image in a living body. This method includes the step of detecting, from a living body, gamma-ray activity generated based on a positron emitted by a compound administered into the living body. The compound is the compound of the present invention. Alternatively, the compound may be provided in the form of the composition of the present invention. In the step of detecting the gamma-ray activity, imaging using a positron emission tomography scanner is generally used. Accordingly, the above method has an advantage of being less invasive to a living body. Such a method can be a data collecting method for assisting physician's diagnosis.

This method is applicable also as a diagnosis method, but, in a case where it is applied to human, it is not intended that the method include a step carried out by a physician (e.g., a step of administering the compound into a living body, a step in which a physician makes judgment). In a case where this method is applied to a non-human mammal, the method may include a step carried out by a veterinarian. In this case, the administration is preferably carried out by injection via various routes, but is not limited to this. Further, a dosage of the compound to be administered can be appropriately designed according to need.

Further, the compound and composition of the present invention can be used for radiotherapy due to its capacity for directing a radioactive metal nuclide to a target tissue in a living body. This indicates that screening for a substance used in radiotherapy is possible. That is, the present invention provides a method for screening for a compound for use in radiotherapy. This method includes the step of detecting, from a living body, gamma-ray activity generated based on a positron emitted by a compound administered into the living body, as in the method for capturing a positron emission tomography image in a living body. The compound is the compound of the present invention. Alternatively, the compound may be provided in the form of the composition of the present invention. In this method, a plurality of types of first polypeptides are prepared according to the above method for producing a compound, these first polypeptides are compared in terms of capacity for directing a radioactive metal nuclide to a target tissue in a living body, and one that is more excellent in the capacity is selected. In this way, intended screening is achieved. In this case, screening accuracy is improved by using a known radiotherapy agent as positive control. In this method, the gamma-ray activity is more preferably detected from a tissue which the radiotherapy is targeted at.

A person skilled in the art who read the present specification easily understands that use of the compound and composition of the present invention that are capable of directing a radioactive metal nuclide to a target tissue in a living body allows a protein and a cell to be successfully labeled.

In one aspect, the present invention provides a method for detecting a protein of interest in a sample. In this method, a sample which contains (or a sample which has a possibility of containing) a protein of interest is incubated with the compound or the composition of the present invention. The protein of interest can be thus successfully radiolabeled (if the protein of interest is present in the sample). This method can be used, for example, as an alternative to a primary antibody in an immunoblot method. As a method for detecting a protein of interest in a sample, various fluorescence labeling techniques are known. Since the compound of the present invention holds a radioactive metal nuclide, the above method makes it possible to detect a protein of interest that is lower in concentration, as compared with the fluorescence labeling techniques.

In another aspect, the present invention provides a method for labeling a cell. In this method, a cell in which a membrane protein of interest is expressed (or a cell which has a possibility that a membrane protein of interest is expressed therein) is incubated with the compound or the composition of the present invention. The membrane protein of interest can be thus successfully radiolabeled (if the membrane protein of interest is present in the cell). This method can be used, for example, as an alternative to a primary antibody in immunohistochemistry.

The present invention provides another method in terms of labeling of a cell. This method includes the steps of: transforming a cell with the use of a polynucleotide encoding a fusion protein between a membrane protein and a second polypeptide bonded to an N-terminus of the membrane protein; and causing a radioactive metal nuclide to be held in a second polynucleotide. See [1-3] above as for the second polypeptide. Use of this method not only allows the polypeptide to be used as a selection marker in the transformation, but also allows a cell that is desired to deliver a radioactive metal nuclide to be given a desired property. In order that the radioactive metal nuclide be bonded by using this method, an amino acid region to which a radioactive metal nuclide is bonded preferably exists outside the cell. A person skilled in the art can easily acquire sequence information of a membrane protein, and therefore can easily construct a membrane protein in which an amino acid region of interest is inserted into a desired site. In a case where the amino acid region of interest constitutes an N-terminus portion of the membrane protein, the amino acid region of interest is created subsequently to a signal peptide that is present in an N-terminus of an immature membrane protein. A procedure for executing these steps is publicly known in the art, and can be carried out by a person skilled in the art without the need for trial and error.

The present invention is not limited to the description of the embodiments above, but may be altered by a skilled person within the scope of the claims. An embodiment based on a proper combination of technical means disclosed in different embodiments is encompassed in the technical scope of the present invention.

All of the academic literatures and patent literatures mentioned in the present specification are incorporated herein by reference.

### [Examples]

The present invention is described below more specifically with reference to the following Examples, but is not limited to these Examples.

### [Example 1]

Octreotide having an N-terminus to which a metal binding sequence was bonded was synthesized by standard Fmoc solid-phase synthesis by using, as a starting material, Fmoc-Thr(tBu)-ol-(2-Cl) trityl resin (0.25 mmol/g). A HAsp(OtBu)-Ala-His(Trt)-Lys(Boc)-D-Phe-Cys(trt)-Phe-Trp-Lys(Boc)-Thr(tBu)-Cys(Trt)-Thr(tBu)-ol-(2-Cl)Trt resin was thus obtained. This resin was washed with methanol, and was then dried under reduced pressure.

50 mg of the resin thus dried was treated for two hours at room temperature with the use of a solution containing 10 µL of triisopropylsilane, 25 µL of ethanedithiol, 25 µL of water, and 940 µL of trifluoroacetic acid. Deprotection and cleavage from the resin were thus performed. After the reaction, a crude peptide was precipitated with the use of 5mL of cold ether. The precipitation was dissolved in an acetonitrile solution and was subjected to reversed-phase HPLC. A fraction of interest was stirred overnight in a PBS buffer to which 5% DMSO was added. This formed a disulfide bond. A crude product was subjected to reversed-phase HPLC. In this way, 2mg of DAHK-Octreotide (H-Asp-Ala-His-Lys-D-Phe-Cys-Phe-Trp-Lys-Thr-Cys-Thr-ol), which was a product of interest, was obtained.

Next, ⁶⁴CuCl₂ was obtained by irradiating ⁶⁴Ni with a proton with the use of a cyclotron by a publicly known method. ⁶⁴CuCl₂ (160 MBq) was reacted with DAHK-Octreotide (0.86 nmol) in 200 µL of a 10mM acetate buffer (pH 6.5) for 1 hour at 40°C. The solution after the reaction was supplied to a C18 solid-phase extraction column, and ⁶⁴Cu-DAHK-Octreotide, which was a product of interest, was dissolved out with ethanol. ⁶⁴Cu-DAHK-Octreotide thus obtained had specific radioactivity of approximately 16 GBq/µmol.

⁶⁴Cu-DAHK-Octreotide (0.2 nmol; approximately 3MBq) thus obtained was dissolved in 200 µl of saline, and the solution thus obtained was intravenously administered to a 10-week-old male Sprague Dawley rat under isoflurane anesthesia. Next, the whole body of this rat was scanned with a continuous bed flow motion by a positron emission tomography scanner (product name microPET Focus220 (produced by SIEMENS)). The scan was conducted during a period of time from 90 minutes later to 120 minutes later from the administration, and an image was reconstructed from obtained signals by an OSEM method.

Fig. 1 shows a PET image of the whole body of the rat. In Fig. 1, the upper arrow indicates the hypophysis, the middle arrow indicates the liver, and the lower arrow indicates the kidney. Fig. 2 shows a PET image of the abdomen of the rat. The arrow in Fig. 2 indicates the pancreas. It was confirmed from these images that ⁶⁴Cu-DAHK-Octreotide accumulated in the hypophysis and the pancreas in which a somatostatin receptor was highly expressed. This level of accumulation was equivalent to a result obtained by using a compound produced with the use of DOTA (the result is not shown). These show that ⁶⁴Cu-DAHK-Octreotide can be used as a radioactive drug.

In a case where a Hepes buffer was used as the buffer used for obtaining ⁶⁴Cu-DAHK-Octreotide, the specific radioactivity could be improved to approximately 50 GBq/µmol. This indicates that the compound of the present invention can be effectively used even in a case where an amount of the compound of the present invention is extremely small.

### [Example 2]

Exenatide having an N-terminus to which a metal binding sequence was bonded was synthesized by standard Fmoc solid-phase synthesis by using, as a starting material, a NovaSyn TGR resin (0.20 mmol/g). A Asp(OtBu)-Ala-His(Trt)-Lys(Boc)-His(Trt)-Gly-Glu(OtBu)-Gly-Thr(tBu)-Phe-Thr(tBu)-Ser(tBu)-Asp(OtBu)-Leu-Ser(tBu)-Lys(Boc)-Gln(Trt)-Met-Glu(OtBu)-Glu(OtBu)-Glu(OtBu)-Ala-Val-Arg(Pbf)-Leu-Phe-Ile-Glu(OtBu)-Trp(Boc)-Leu-Lys(Boc)-Asn(Trt)-Gly-Gly-Pro-Ser(tBu)-Ser(tBu)-Gly-Ala-Pro-Pro-Pro-Ser(tBu)-NovaSyn TGR resin was thus obtained. This resin was washed with methanol, and was then dried under reduced pressure.

50 mg of the resin thus dried was treated for two hours at room temperature with the use of a solution containing 10 µL of triisopropylsilane, 25 µL of ethanedithiol, 25 µL of water, and 940 µL of trifluoroacetic acid. Deprotection and cleavage from the resin were thus performed. After the reaction, a crude peptide was precipitated with the use of 5mL of cold ether. The precipitation was dissolved in an acetonitrile solution and was subjected to reversed-phase HPLC. In this way, 4mg of DAHK-Exenatide (Asp-Ala-His-Lys-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH₂), which was a product of interest, was obtained.

Next, ⁶⁴CuCl₂ was obtained by irradiating ⁶⁴Ni with a proton with the use of a cyclotron by a publicly known method. ⁶⁴CuCl₂ (174 MBq) was reacted with DAHK-Exenatide (0.75 nmol) in 100 µL of a 10mM acetate buffer (pH 6.5) for 1 hour at 40°C. The solution after the reaction was supplied to a C18 solid-phase extraction column, and ⁶⁴Cu-DAHK-Exenatide, which was a product of interest, was dissolved out with ethanol. ⁶⁴Cu-DAHK-Exenatide thus obtained had specific radioactivity of approximately 80 GBq/µmol.

⁶⁴Cu-DAHK-Exenatide (0.1 nmol; approximately 14 MBq) thus obtained was dissolved in 200 µl of saline, and the solution thus obtained was intravenously administered to a tail vein of a 36-week-old male Bulb/c nude mouse under isoflurane anesthesia. Next, the whole body of this mouse was scanned by a positron emission tomography scanner (product name microPET Focus220 (produced by SIEMENS)). The scan was conducted during a period of time from immediately after the administration to 90 minutes later from the administration, and an image was reconstructed from obtained signals by an FBP method.

Fig. 3 shows a PET image of the whole body of the mouse. In Fig. 3, the arrow (1) indicates the pancreas, the arrow (2) indicates the kidney, the arrow (3) indicates the liver, and the arrow (4) indicates the bladder. It was confirmed from these images that ⁶⁴Cu-DAHK-Exenatide accumulated in the pancreas in which a GLP-1 receptor was highly expressed.

### Industrial Applicability

The present invention is useful for diagnosis, judgment, and treatment of diseases, and therefore greatly contributes to the medical field.

## Claims

1. A compound comprising:
a first polypeptide or an analogue thereof; and
a second polypeptide bonded to an N-terminus of the first polypeptide or the analogue thereof,
the first polypeptide being a polypeptide that specifically binds with a protein expressed in a target tissue,
the second polypeptide having an amino acid region that has a high affinity for a radioactive metal nuclide, and
the radioactive metal nuclide that has a high affinity for the amino acid region being held in the amino acid region.

2. The compound according to claim 1, wherein the amino acid region is a fragment of a naturally-occurring protein that exists in a living body to which the compound is applied.

3. The compound according to claim 1 or 2, wherein the amino acid region contains X1-His where X1 represents any amino acid other than Pro.

4. The compound according to any one of claims 1 through 3, wherein the amino acid region contains X2-X1-His or X2-X1-His-Lys where X1 represents any amino acid other than Pro and X2 represents Met, Asp or Glu.

5. The compound according to any one of claims 1 through 4, wherein the amino acid region contains Met-X3-Cys-X4-X5-Cys where X3 represents Thr, Ser, His or Asn, X4 represents Ala, Asn, Gln, Ser, Asp, Gly or Glu, and X5 represents Gly, Ser, Ala, His, Thr, Asn, Gln or Glu.

6. The compound according to claim 1 or 2, wherein the amino acid region contains His-Gly-Asp-His-Met-His-Asn-His-Asp-Thr-Lys.

7. The compound according to any one of claims 1 through 6, wherein the amino acid region constitutes an N-terminus portion of the second polypeptide.

8. The compound according to any one of claims 1 through 7, wherein the second polypeptide is made up of 3 to 15 amino acids.

9. The compound according to any one of claims 1 through 8, wherein the radioactive metal nuclide held in the amino acid region is ⁶⁴Cu.

10. The compound according to any one of claims 1 through 9, wherein the protein is a receptor protein for the first polypeptide.

11. A method for producing a compound, comprising the step of bonding a second polypeptide to an N-terminus of a first polypeptide or an analogue thereof,
the first polypeptide being a polypeptide that specifically binds with a protein expressed in a target tissue,
the second polypeptide having an amino acid region that has a high affinity for a radioactive metal nuclide, and,
the method further comprising the step of causing the radioactive metal nuclide that has a high affinity for the amino acid region to be held in the amino acid region.

12. The method according to claim 11, wherein the step of bonding the second polypeptide is carried out by synthesizing the second polypeptide to the N-terminus of the first polypeptide or the analogue thereof by solid-phase peptide synthesis.

13. The method according to claim 11, wherein the step of bonding the second polypeptide is carried out by generating a fusion protein between the first polypeptide and the second polypeptide by a genetic engineering method.

14. A composition for allowing a radioactive metal nuclide to direct to a target tissue in a living body, comprising a compound,
the compound having:
a first polypeptide or an analogue thereof; and
a second polypeptide bonded to an N-terminus of the first polypeptide or the analogue thereof,
the first polypeptide being a polypeptide that specifically binds with a protein expressed in a target tissue, and
the second polypeptide having an amino acid region that has a high affinity for the radioactive metal nuclide.

15. The composition according to claim 14, wherein the composition is used for diagnosis using positron emission tomography.

16. The composition according to claim 14, wherein the composition is used for radiotherapy.

17. The composition according to any one of claims 14 through 16, further comprising the radioactive metal nuclide that has a high affinity for the amino acid region.

18. A method for capturing a positron emission tomography image in a living body,
the method comprising the step of detecting, from a living body, gamma-ray activity generated based on a positron emitted by a compound administered into the living body,
the compound having a first polypeptide or an analogue thereof, and a second polypeptide bonded to an N-terminus of the first polypeptide or the analogue thereof, and
in the compound, the first polypeptide being a polypeptide that specifically binds with a protein expressed in a target tissue to be measured, the second polypeptide having an amino acid region that has a high affinity for a radioactive metal nuclide, and the radioactive metal nuclide that has a high affinity for the amino acid region being held in the amino acid region.

19. A method for screening for a compound for use in radiotherapy,
the method comprising the step of detecting, from a living body, gamma-ray activity generated based on a positron emitted by a compound administered into the living body,
the candidate compound having a first polypeptide or an analogue thereof, and a second polypeptide bonded to an N-terminus of the first polypeptide or the analogue thereof,
in the candidate compound, the first polypeptide being a polypeptide that specifically binds with a protein expressed in a target tissue to be measured, the second polypeptide having an amino acid region that has a high affinity for a radioactive metal nuclide, and the radioactive metal nuclide that has a high affinity for the amino acid region being held in the amino acid region.

20. A method for detecting a protein of interest in a sample, comprising the steps of:
incubating the sample with a compound; and
detecting gamma-ray activity generated based on a positron emitted from the compound,
the compound having a first polypeptide or an analogue thereof, and a second polypeptide bonded to an N-terminus of the first polypeptide or the analogue thereof,
in the compound, the first polypeptide being a polypeptide that specifically binds with a membrane protein of interest, the second polypeptide having an amino acid region that has a high affinity for a radioactive metal nuclide, and the radioactive metal nuclide that has a high affinity for the amino acid region being held in the amino acid region.

21. A method for labeling a cell, comprising the steps of:
incubating the cell with a compound; and
detecting gamma-ray activity generated based on a positron emitted from the compound,
the compound having a first polypeptide or an analogue thereof, and a second polypeptide bonded to an N-terminus of the first polypeptide or the analogue thereof,
in the compound, the first polypeptide being a polypeptide that specifically binds with a membrane protein expressed in the cell to be labeled, the second polypeptide having an amino acid region that has a high affinity for a radioactive metal nuclide, and the radioactive metal nuclide that has a high affinity for the amino acid region being held in the amino acid region.

22. A method for labeling a cell, comprising the steps of:
transforming the cell with use of a polynucleotide encoding a fusion protein between a membrane protein and a second polypeptide bonded to an N-terminus of the membrane protein; and
causing a radioactive metal nuclide to be held in a second polynucleotide,
the second polypeptide having an amino acid region that has a high affinity for the radioactive metal nuclide.
